Europäisches Patentamt

European Patent Office

Office européen des brevets

(11) Numéro de publication: **0 343 085**
**A1**

(12) **DEMANDE DE BREVET EUROPEEN**

(21) Numéro de dépôt: **89401388.7**

(22) Date de dépôt: **19.05.89**

(51) Int. Cl.⁴: **C 07 K 7/06**
**A 61 K 37/02**

(30) Priorité: **20.05.88 FR 8806790**

(43) Date de publication de la demande:
**23.11.89 Bulletin 89/47**

(84) Etats contractants désignés:
**BE CH DE FR GB IT LI NL**

(71) Demandeur: **CENTRE NATIONAL DE LA RECHERCHE SCIENTIFIQUE (CNRS)**
**15, Quai Anatole France**
**F-75007 Paris (FR)**

(72) Inventeur: **Jolles, Pierre,**
**2, rue Jean-François Gerbillon,**
**F-75006 Paris (FR)**

**Fiat, Anne-Marie, née Duizabo,**
**67 bis, avenue des Pages,**
**F-78110 Le Vesinet (FR)**

**Lévy, Sylviane, née Toledano,**
**16, rue des Sablons,**
**F-75116 Paris (FR)**

**Drouet, Ludovic,**
**14, avenue des Cottages,**
**F-92340 Bourg-la-Reine (FR)**

(74) Mandataire: **Ayache, Monique**
**CABINET AYACHE ET AUTRES 3 Côte de la Jonchère**
**B.P. 24**
**F-92502 Rueil Malmaison (FR)**

(54) **Nouveaux pentapeptides et leurs applications en tant que médicaments, notamment antithrombostiques.**

(57) Les pentapeptides selon l'invention répondent à la formule :
lysyl-X-glutaminyl-aspartyl-lysine (I)
dans laquelle X représente asparaginyl ou isoleucyl.

Application en médecine, notamment dans la prévention et le traitement des thromboses.

EP 0 343 085 A1

## Description

**Nouveaux pentapeptides et leurs applications en tant que médicaments, notamment antithrombotiques.**

La présente invention a été faite dans le cadre d'une collaboration entre le Laboratoire ASPECTS STRUCTURAUX (GENES ET PROTEINES) ET FONCTION DE SUBSTANCES BIOLOGIQUEMENT ACTIVES de l'Université PARIS V et le Laboratoire PLAQUETTES ET HEMOSTASES de l'Université PARIS VII, laboratoires associés au C.N.R.S..

Elle a pour objet de nouveaux pentapeptides à activité antithrombotique.

Plus précisément, l'invention a pour objet les pentapeptides Lys-X-Gln-Asp-Lys, X pouvant représenter Asn ou Ile, ou, en désignant les amino-acides au moyen du système d'abréviations à une lettre, les pentapeptides KXQDK, X pouvant représenter N ou I, et leurs applications thérapeutiques, notamment en tant qu'agents antithrombotiques.

Pierre Jollès et Agnès Henschen [TIBS 7 (1982) 325-328] ont noté plusieurs analogies entre les phénomènes de coagulation du sang et du lait. C'est ainsi, par exemple, que des homologies entres les séquences peptidiques de la chaîne γ du fibrinogène humain et de la caséine κ de vache ont été mises en évidence [P. Jollès, M.-H. Loucheux-Lefebvre et A. Henschen, J. Mol. Evol. 11 (1978) 271-277].

Quelques peptides provenant du fibrinogène inhibent l'agrégation plaquettaire. Il s'agit notamment du dodécapeptide correspondant à l'enchaînement C-terminal de la chaîne γ du fibrinogène (résidus 400→411) et de peptides contenant la séquence RGD commune à des séquences de la chaîne α du fibrinogène, du facteur de von Willebrand (VWF) et de la fibronectine.

Un grand nombre de données accumulées ces dernières années est en faveur du fait que le récepteur du fibrinogène est situé sur un complexe de deux glycoprotéines plaquettaires GPIIb et GPIIIa situées sur la membrane plasmatique des plaquettes. La liaison du fibrinogène au récepteur plaquettaire nécessite l'activation des plaquettes par des agonistes tels que l'ADP, la thrombine, etc., ce qui semble avoir pour effet d'exposer les sites de liaison du fibrinogène (voir par exemple W.M. Isenburg et coll. dans Blood, Novembre 1986, n° 1150).

Récemment P. Jollès et coll. [voir Eur. J. Biochem. 158 (1986) 379-382] ont trouvé qu'un undécapeptide de la caséine κ de vache, naturel ou synthétique, inhibe à la fois l'agrégation plaquettaire et la liaison du fibrinogène.

Ceci étant, les inventeurs, poursuivant les investigations entreprises dans ce domaine, ont eu l'idée de rechercher le (ou les) peptide(s) le(s) plus court(s) possible ayant une action similaire.

C'est ainsi qu'ils ont eu l'idée de synthétiser deux pentapeptides nouveaux, à savoir le pentapeptide Lys-Asn-Gln-Asp-Lys ou KNQDK qui correspond à la séquence 112→116 de la caséine κ de lait de vache, et le pentapeptide Lys-Ile-Gln-Asp-Lys ou KIQDK qui, lui, correspond à la séquence 100→104 de la caséine κ de lait humain, et d'étudier leur activité pharmacologique.

Selon l'un de ses aspects, l'invention a donc pour objet un pentapeptide répondant à la formule:
lysyl-X-glutaminyl-aspartyl-lysine (I)
dans laquelle X représente asparaginyl ou isoleucyl.

Les pentapeptides répondant à cette formule peuvent être obtenus par synthèse peptidique classique ou, plus simplement, en ayant recours à un synthétiseur de peptides.

Ces pentapeptides sont capables d'inhiber l'agrégation plaquettaire et la liaison du fibrinogène sur des plaquettes stimulées, ainsi que la formation des thromboses.

Compte tenu de ces propriétés, ces peptides peuvent être utilisés en thérapeutique, notamment dans la prévention et le traitement des thromboses.

Ils peuvent notamment être administrés par injection intra-musculaire, notamment en solution aqueuse physiologique, par instillation nasale, ou encore par voie orale, notamment sous forme de gouttes.

L'exposé qui suit est destiné à mieux expliquer l'invention.

### Synthèse et purification du peptide KNQDK

Ce peptide a été synthétisé à l'aide du synthétiseur de peptides de la société Applied Biosystems commercialisé sous la référence 430A, selon la méthode de Merrifield.

Il a ensuite été purifié par chromatographie liquide haute performance (HPLC) dans les conditions suivantes :

Colonne : de 4,5 x 300 mm, silice greffée, phase inverse, porosité 100 Å, diamètre des particules 5 μ, commercialisée sous la dénomination Nucleosil® C 18 par la société MACHEREY NAGEL.

Eluant : eau/acétonitrile/acide trifluoroacétique dans les proportions volumétriques 970:30:1.

Débit: 1 ml/min.

Lecture à 215 nm.

Sortie du pic à 9,50 min.

Il a ensuite été lyophilisé.

### Analyse

Après hydrolyse totale par HCl 6N + mercapto-2 éthanol au 1/2000è, pendant 18 heures à 110°C sous vide, on a trouvé que la composition en acides aminés (résidus/mole) était la suivante :

D : 1,80
E : 1,0
K : 2,3
en utilisant un appareil Biotronik®, modèle LC 6000 commercialisé par la société SEMSA.

La séquence du peptide a été établie à l'aide du séquenceur de la société Applied Biosystems, référence 470A.

Les phénylthiohydantoïnes (PTH) des acides aminés ont été identifiées par HPLC avec le chromatographe de la société Applied Biosystems référence 120A.

La séquence trouvée est la suivante :

K    N    Q    D    K    *
→    →    →    →    →

*→    : amino acide déterminé par dégradation automatique
          d'Edman.

Aucune trace d'impureté n'a pu être décelée. Ce peptide est donc pur à 99 % au moins.

Caractéristiques physiques :
Ce peptide est hydrophile et hygroscopique.

Synthèse et purification du peptide KIQDK
Ce peptide a été synthétisé à l'aide du synthétiseur du peptides de la société Applied Biosystems commercialisé sous la référence 430A suivant la méthode de Merrifield.

Il a ensuite été purifié par chromatographie liquide haute performance (HPLC) dans les conditions suivantes :
Colonne : de 3,9 X 300 mm de silice greffée, phase inverse, porosité 125Å, diamètre des particules 10 μ, commercialisée sous la dénomination μ Bondapak® C 18 par la société WATERS CHROMATOGRAPHY.
Solvant de départ : $H_2O$/acétonitrile/acide trifluoroacétique : 989/10/1
Gradient en acétonitrile : 1 à 16 % en 25 min.
Débit : 2 ml/min.
Lecture à 210 nm.
Sortie du pic à 6,4 min.
Il a ensuite été lyophilisé.

Analyse
Après hydrolyse totale par HCl 6N + mercapto-2 éthanol au 1/2000è, pendant 18 heures à 110°C sous vide, on a trouvé que la composition en acides aminés (résidus/mole) était la suivante :
D : 1,0
E : 1,05
I : 1,0
K : 1,97
en utilisant un appareil Biotronik®, modèle LC 6000 commercialisé par la société SEMSA.

La séquence du peptide a été établie à l'aide du séquenceur de la société Applied Biosystems, référence 470A.

Les phénylthiohydantoïnes (PTH) des acides aminés ont été identifiées par HPLC avec le chromatographe de la société Applied Biosystems référence 120A.

La séquence trouvée est la suivante :

K    I    Q    D    K    *
→    →    →    →    →

*→    : amino acide déterminé par dégradation automatique
          d'Edman.

Aucune trace d'impureté n'a pu être décelée. Ce peptide est donc pur à 99 % au moins.

Caractéristiques physiques :
Ce peptide est hydrophile et hygroscopique.

Etude pharmacologique :

1 -Etude pharmacologique in vitro sur les fonctions plaquettaires.

a) plaquettes humaines.

On a effectué une étude des pentapeptides KNQDK et KIQDK sur les fonctions plaquettaires à savoir : l'agrégation des plaquettes et la liaison du fibrinogène induites par l'ADP. En effet, il est maintenant clairement établi que le site de liaison du fibrinogène se trouve sur le complexe GPIIb/IIIa de la membrane plaquettaire : une inhibition de la liaison laisserait à penser que ces peptides agissent à ce niveau, soit directement, soit indirectement.

Dans le cas du peptide KNQDK, il a ainsi été trouvé qu'une concentration 500 µM de ce peptide inhibe 50 % de l'agrégation plaquettaire induite par l'ADP 10 µM, tandis qu'une inhibition de 50 % de la liaison du fibrinogène aux plaquettes est obtenue avec une concentration 200 µM de ce peptide.

Dans le cas du pentapeptide KIQDK, il faut une concentration beaucoup plus élevée pour obtenir 50 % d'inhibition de la liaison du fibrinogène soit 1100 µM, tandis qu'une concentration 350 µM suffit pour obtenir 50 % d'inhibition de l'agrégation des plaquettes.

La modification de N en I semble donc affecter la liaison du fibrinogène.

Le KNQDK est donc un inhibiteur de l'agrégation plaquettaire à l'ADP chez l'homme.

Il a été de plus étudié :
- si le KNQDK est aussi un inhibiteur de l'agrégation de plaquettes de rat et de cobaye sur plaquettes isolées ;
- si cet effet in vitro était corrélé à un effet antithrombotique in vivo.

b) Plaquettes animales
Le sang est recueilli sur citrate de sodium à 0,13 mol/l, à raison de 1v/9v pour les plaquettes de cobaye et de 0,5 v/9,5v pour les plaquettes de rat . Les plaquettes suspendues dans leur propre plasma (plasma enrichi en plaquettes ou PRP) sont isolées par centrifugation.

L'agrégation plaquettaire est induite par l'ADP 1,2 µM. La préincubation des plaquettes avec le KNQDK (0,85 à 10 µM) induit une inhibition qui est dépendante de la dose, à savoir :
- chez le rat :
$CI_{50} = 4 \times 10^{-6}$ M et 100 % d'inhibition pour une concentration $C = 10^{-5}M$.
- chez le cobaye :
$CI_{50} = 6 \times 10^{-6}$ M et 100 % d'inhibition pour une concentration $C = 10^{-5}M$.

A titre de comparaison, le RGDS, un autre peptide du fibrinogène connu pour cet effet chez l'homme :
- n'induit pas d'inhibition significative chez le rat (moins de 40 % d'inhibition pour une concentration $C = 4 \times 10^{-3}M$.)
- induit une inhibition chez le cobaye mais pour des concentrations beaucoup plus élevées, à savoir :
$CI_{50} = 1,5 \times 10^{-4}$ M et 100 % d'inhibition pour une concentration $C = 1,5 \times 10^{-3}$ M.

2 - Etude pharmacologique in vivo.
L'effet observé in vitro a été corrélé à un effet anti-thrombotique in vivo.

Selon le modèle utilisé, on créé une lésion endothéliale localisée sur une artériole du mésentère chez le rat ou le cobaye. Un thrombus se développe d'une manière cyclique. La quantification de la thrombose repose sur le nombre de thromboses formées pendant 15 minutes d'observation.

L'injection intra-veineuse de KNQDK entraîne une inhibition du nombre de thromboses qui est dépendante de la dose, à savoir :
- chez le rat, 50 % d'inhibition pour une dose de 8 µM/kg
- chez le cobaye, 50 % d'inhibition pour une dose de 6 µM/kg.

Cet effet dure environ 30 minutes et le retard d'apparition de la thrombose est multiplié par 10 par rapport au RGDS.

A titre de comparaison, le RGDS entraîne une inhibition du nombre de thromboses qui est dépendante de la dose, à savoir :
- chez le rat 50 % d'inhibition pour une dose de 2 µM/kg
- chez le cobaye 50 % d'inhibition pour une dose de 1 µM/kg.

Cet effet dure au moins 75 minutes.

Les résultats obtenus par le demandeur suggèrent qu'alors que le RGDS a un effet anti-thrombotique non relié à l'activité anti-agrégante plaquettaire, le KNQDK a un effet anti-thrombotique qui semble dépendre de l'activité anti-agrégante plaquettaire.

## Revendications

1. Pentapeptide répondant à la formule :
lysyl-X-glutaminyl-aspartyl-lysine (I)
dans laquelle X représente asparaginyl ou isoleucyl.

2. Le pentapeptide lysyl-asparaginyl-glutaminyl-aspartyl-lysine.

3. Le pentapeptide lysyl-isoleucyl-glutaminyl-aspartyl-lysine.

4. Médicament caractérisé en ce qu'il contient, en tant que principe actif, au moins l'un des pentapeptides selon la revendication 1.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | CHEMICAL ABSTRACTS, vol. 105, 1986, page 427, résumé no. 95118t, Columbus, Ohio, US; P. JOLLES et al.: "Analogy between fibrinogen and casein. Effect of an undecapeptide isolated from k-casein on platelet function", & EUR. J. BIOCHEM. 1986, 158(2), 379-82 --- | | C 07 K 7/06<br>A 61 K 37/02 |
| A | EP-A-0 220 957 (SCRIPPS CLINIC AND RESEARCH FOUNDATION) ----- | | |

DOMAINES TECHNIQUES RECHERCHES (Int. Cl.4)

C 07 K
A 61 K

Le présent rapport a été établi pour toutes les revendications

| Lieu de la recherche | Date d'achèvement de la recherche | Examinateur |
|---|---|---|
| LA HAYE | 23-08-1989 | NOVOA Y SANJURJO M.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)